# EUROPEAN PATENT APPLICATION

(11) **EP 2 556 789 A1**
(43) Date of publication of application: **13.02.2013**
(21) Application number: 12005787.2
(22) Date of filing: 09.08.2012
(51) Int. Cl.: A61B 1/005

(54) **Endoscope**

(30) Priority: 10.08.2011 JP 2011175315
(71) Applicant: Olympus Corporation, Shibuya-ku Tokyo (JP)
(72) Inventor: Kato, Takahiko, Tokyo 151-0072 (JP)
(74) Representative: Schicker, Silvia

(57) **Abstract**

An endoscope of the present invention includes a cover member 10, a groove 11 formed on an outer circumferential face 10g of the cover member 10, holes 25 and 26 which are located at least two places in a circumferential direction of the groove 11 and communicate with the interior of the cover member 10, a bending portion 3b and a loop-shaped puller wire 50 wound around the groove 11 so as to enter the groove 11 from within the cover member 10 through the hole 25 at one end in the cover member 10 and enter the cover member 10 from the groove 11 through the hole 26 at the other end.

## Description

### BACKGROUND OF THE INVENTION

### 1. Field of the Invention

The present invention relates to an endoscope provided with an insertion portion which is inserted into an object.

### 2. Description of the Related Art

Whether insertability of the insertion portion of the endoscope with respect to the object is good or bad is greatly affected by an outside diameter of the insertion portion, a length of a distal end portion in the insertion direction which is located on the insertion portion on a distal end side in an insertion direction (hereinafter, simply referred to as "distal end side") or the like.

Endoscopes used in an industrial field in particular are mostly used to inspect an object whose inner wall is made of a rigid material such as metal, and as such, if the outside diameter of the insertion portion is larger even by 0.1 mm, the insertion portion cannot be inserted into the object or if the distal end portion is long in the insertion direction, the insertion portion may not be able to pass through bent portions in the object.

Here, endoscopes whose insertion portion includes a bending portion are known to have a configuration in which a puller wire for bending the bending portion toward a plurality of directions is inserted in the insertion portion.

The distal end of the puller wire in the insertion direction (hereinafter simply referred to as "distal end") is fixed to an inner surface of a bending piece located closest to the distal end side out of a plurality of bending pieces constituting the bending portion and the bending portion is configured to be bent by pulling the puller wire from the operation section side.

However, if the distal end of the puller wire is fixed to the inner surface of the bending piece of the outermost distal end, the inner space becomes narrower by an amount corresponding to the fixed portion, resulting in a problem that the outside diameter of the insertion portion cannot help but be increased to secure the inner space.

Thus, Japanese Patent Application Laid-Open Publication No.62-227313 discloses a configuration in which a bending piece located at the outermost distal end is coated and fixed to the outer circumference of a distal end rigid portion on the proximal end side in the insertion direction constituting the distal end portion of the insertion portion (hereinafter simply referred to as "proximal end side"), a puller wire on the distal end side is set in a groove formed along the insertion direction on the outer circumference of the distal end rigid portion, and the distal end of the puller wire is then bent at a substantially right angle and fixed to a through hole formed in the bending piece located at the outermost distal end so that it is possible to secure a wide inner space for the insertion portion on the distal end side without increasing the outside diameter of the insertion portion.

Furthermore, Japanese Patent Application Laid-Open Publication No.2001-37705 discloses a configuration in which a groove is provided on the outer circumference of a distal end cover of the distal end portion on the proximal end side along a circumferential direction of the distal end cover, a puller wire drawn from within the insertion portion is wound around the groove, the puller wire is caught in and fixed to the groove using a screw, the groove is coated with bending portion coating rubber for coating the outer circumference of the bending piece so that the bending portion coating rubber prevents the puller wire fixed to the groove from being rusted by water content or the like that enters the groove and a wide inner space can be secured for the insertion portion on the distal end side without increasing the outer diameter of the insertion portion.

However, the configuration of Japanese Patent Application Laid-Open Publication No.62-227313 adopts the configuration in which the outer circumference of the bending piece is covered with a covering tube to secure watertightness of the bending portion, resulting in a problem that the outside diameter of the insertion portion in the region where the puller wire is fixed tends to increase by an amount corresponding to the covering tube.

Furthermore, in the configuration of Japanese Patent Application Laid-Open Publication No.2001-37705, in order to prevent the diameter of the insertion portion from increasing and prevent a height difference from being formed between the distal end cover and the bending portion coating rubber on the outer circumferential face of the insertion portion, the proximal end side of the distal end cover where the distal end side of the bending portion coating rubber is coated is placed embedded in the inside in the diameter direction, and further a thickness of a region where the screw is fixed needs to be secured on the distal end cover proximal end side, causing the inner space on the cover proximal end side to become narrower.

When the inner space becomes narrower, the number of known light guides inserted in the insertion portion decreases, which causes the light quantity of illuminating light to decrease, and the diameter of an image pickup cable inserted in the insertion portion cannot help but be decreased, resulting in problems such that image noise increases.

Furthermore, in the configuration of Japanese Patent Application Laid-Open Publication No.2001-37705, the distal end of the bending portion coating rubber needs to be fixed at a position other than the position of the groove which is wound with the puller wire or the screw, resulting in a problem that the length of the distal end portion tends to increase.

The present invention has been implemented in view of the aforementioned problems and it is an object of the present invention to provide an endoscope having a configuration that allows a distal end portion thereof to be shortened while reducing the diameter of the insertion portion distal end side and securing a sufficient inner space.

### SUMMARY OF THE INVENTION

In summary, an endoscope according to the present invention includes a cover member that constitutes a distal end portion on a distal end side in an insertion direction of an insertion portion inserted into an object and covers inner components in the distal end portion, a groove formed along a circumferential direction of the cover member on an outer circumferential face of the cover member, holes located in at least two places in the circumferential direction of the groove, communicating with an interior of the cover member and formed in the cover member, a bending portion located behind the distal end portion in the insertion direction of the insertion portion, having a bent tube and a soft tube member for coating the bent tube in which the distal end side of the bent tube in the insertion direction is engaged within a proximal end of the cover member in the insertion direction and the distal end of the soft tube member in the insertion direction contacts the proximal end of the cover member, and a puller wire that has an outside diameter equal to or smaller than a depth of the groove, is inserted into the insertion portion so as to bend the bending portion in a plurality of directions, enters the groove from the interior of the cover member through one of the holes of the cover member, is folded into a loop shape so as to enter the cover member through the other hole from the groove and is wound around the groove.

The above and other objects, features and advantages of the invention will become more clearly understood from the following description referring to the accompanying drawings.

### BRIEF DESCRIPTION OF THE DRAWINGS

Fig. 1 is a perspective view illustrating an endoscope system provided with an endoscope of a first embodiment;
Fig. 2 is an enlarged perspective view illustrating a distal end side of an insertion portion of the endoscope in Fig. 1;
Fig. 3 is a cross-sectional view of the insertion portion on the distal end side along a line III-III in Fig. 2;
Fig. 4 is a cross-sectional view of the distal end portion along a line IV-IV in Fig. 3;
Fig. 5 is an enlarged perspective view of a configuration of a modification example of the insertion portion of the endoscope on the distal end side in Fig. 2;
Fig. 6 is a cross-sectional view of the insertion portion on the distal end side along a line VI-VI in Fig. 5;
Fig. 7 is a cross-sectional view of the distal end portion along a line VII-VII in Fig. 6;
Fig. 8 is an enlarged partial cross-sectional view illustrating the engagement region between the distal end portion and the bending portion in the insertion portion of an endoscope according to a second embodiment;
Fig. 9 is an enlarged plan view of the distal end side of the bent tube in Fig. 8;
Fig. 10 is a partial cross-sectional view of the engagement region with the puller wire and adhesive omitted from Fig. 8;
Fig. 11 is an enlarged perspective view illustrating the insertion portion on the distal end side in a configuration according to a modification example where a puller wire is wound around the bent tube;
Fig. 12 is a side view of the distal end portion in Fig. 11 viewed from a direction XII in Fig. 12;
Fig. 13 is a cross-sectional view of the insertion portion on the distal end side along a line XIII-XIII in Fig. 12;
Fig. 14 is a cross-sectional view of the insertion portion on the distal end side along a line XIV-XIV in Fig. 12; and
Fig. 15 is a cross-sectional view illustrating a modification example where the puller wire is set in a plurality of holes of the groove of the cover member in Fig. 4 in a running stitch form.

### DETAILED DESCRIPTION OF THE PREFERRED EMBODIMENTS

Hereinafter, embodiments of the present invention will be described with reference to accompanying drawings.

### (First embodiment)

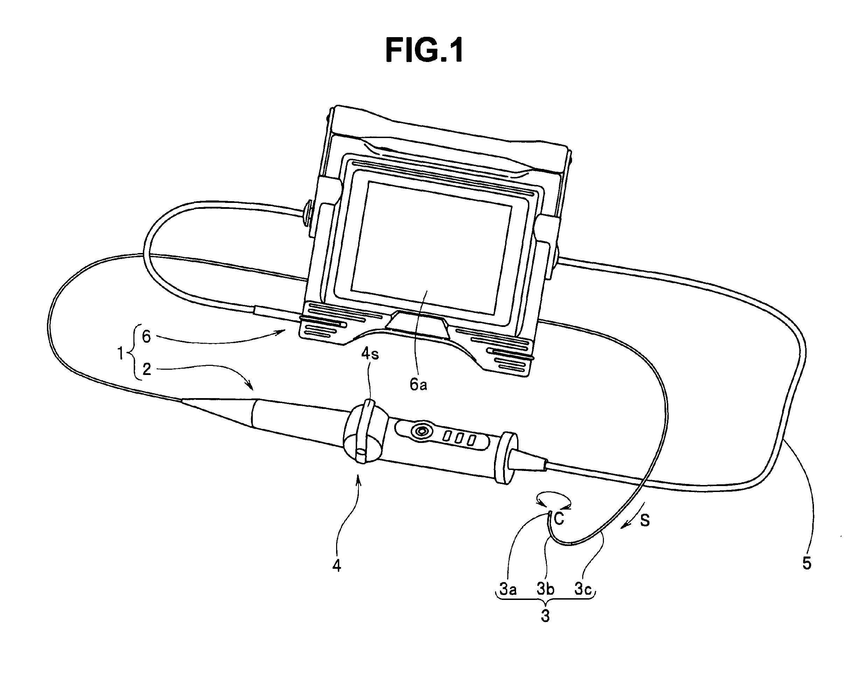

Fig. 1 is a perspective view illustrating an endoscope system provided with an endoscope of the present embodiment.

As shown in Fig. 1, principal parts of an endoscope system 1 are configured by including an endoscope 2 and a main unit 6.

Principal parts of the endoscope 2 are configured by including an elongated insertion portion 3 which is inserted into an object, an operation section 4 provided on the proximal end side of the insertion portion 3 and a universal cord 5 that extends from the operation section 4.

Principal parts of the insertion portion 3 is configured by including a distal end portion 3a, a bending portion 3b that is configured to be bendable, for example, in a vertical direction and a flexible tube portion 3c that is a long flexible portion, in order from the distal end side.

The operation section 4 is formed in a cylindrical shape and is provided with a bending operation lever 4s to cause the bending portion 3b to perform bending operation.

The bending portion 3b is configured to bend, for example, in an upward direction or downward direction according to pivoting operation of the bending operation lever 4s.

Furthermore, in addition to the bending operation lever 4s, the operation section 4 is provided with a switch for instructing image pickup operation or the like.

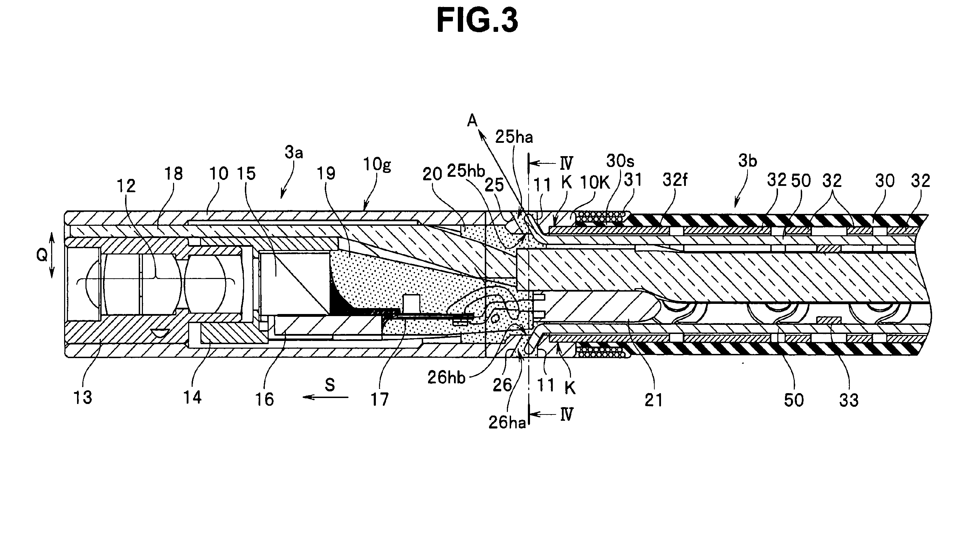

Furthermore, a light source such as a light-emitting element that supplies illuminating light to the interior of an object from the front end face of the distal end portion 3a via a light guide 18 (see Fig. 3) which will be described later and a pulley around which a puller wire 50 (see Fig. 3) which will be described later is wound or the like are provided inside the operation section 4.

The extending end of the universal cord 5 is connected to the main unit 6. A signal cable 21 (see Fig. 3) which extends from the main unit 6, and a distal end of which is connected to an image pickup device 16 (see Fig. 3) which is provided in the distal end portion 3a and which will be described later and an electric wire or the like that extends from the main unit 6 to supply power to a light-emitting element provided in the operation section 4 or the like are inserted into the universal cord 5.

The main unit 6 is provided with a monitor 6a that displays an endoscope image picked up by an image pickup device 16 which is provided in the distal end portion 3a and will be described later.

Furthermore, a CPU for image processing, electric parts such as a recording apparatus for recording processed images and a battery unit that supplies power, or the like are provided in the main unit 6.

Power of the battery is supplied to the monitor 6a, the image pickup device 16 in the distal end portion 3a, a light-emitting element provided in the operation section 4 and the aforementioned electric parts or the like.

A case has been described in the aforementioned embodiment where the main unit 6 and the universal cord 5 are connected together, but the configuration is not limited to this, and for example, a configuration may be adopted in which a connector (including USB or the like) is provided at the extending end of the universal cord 5 so that the connector is detachably connected to the main unit 6.

Furthermore, in the case of the configuration in which the connector is provided at the extending end of the universal cord 5, the main unit 6 may be substituted by a personal computer (PC).

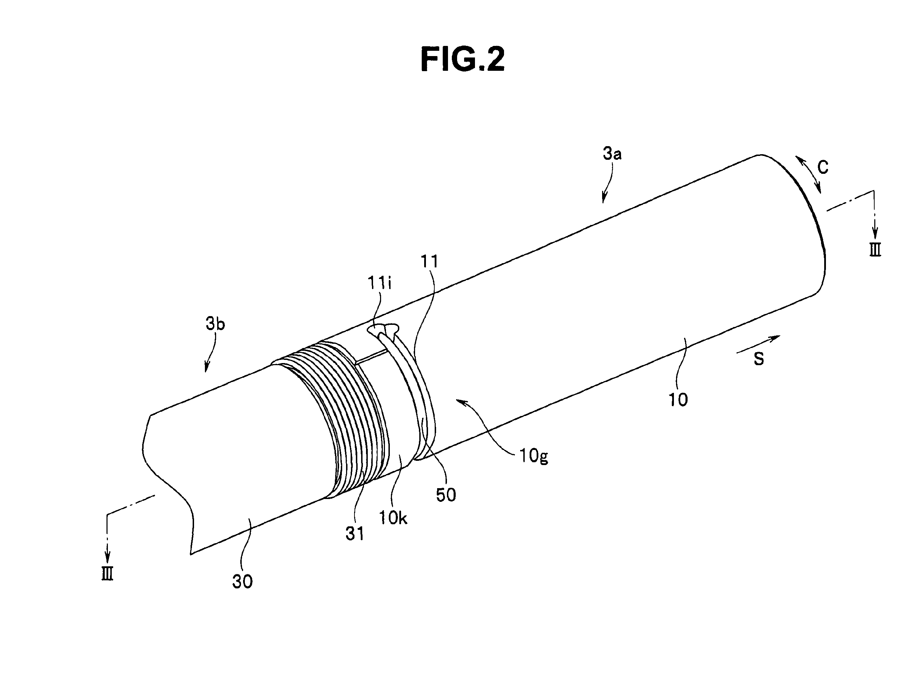

Next, an internal configuration of the insertion portion 3 on the distal end side will be described using Fig. 2 to Fig. 4.

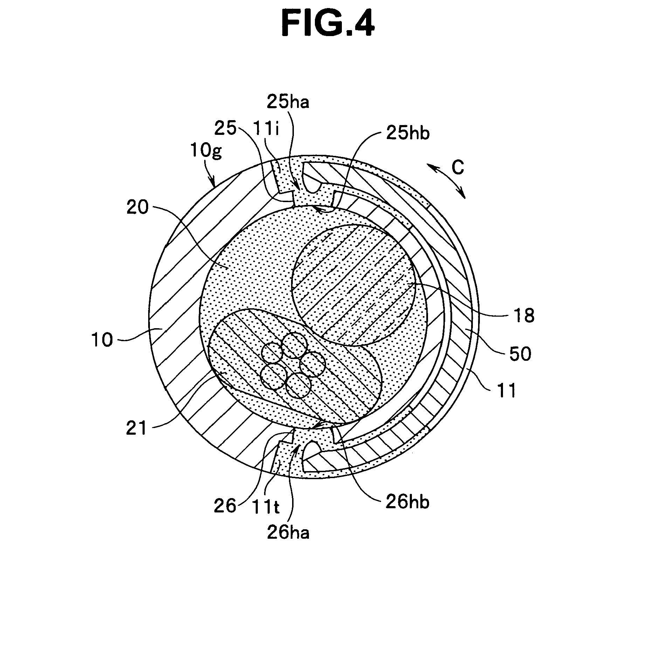

Fig. 2 is an enlarged perspective view illustrating the insertion portion on the distal end side of the endoscope in Fig. 1, Fig. 3 is a cross-sectional view of the insertion portion on the distal end side along a line III-III in Fig. 2, and Fig. 4 is a cross-sectional view of the distal end portion along a line IV-IV in Fig. 3,

As shown in Fig. 2 to Fig. 4, the distal end portion 3a is provided with a cover member 10 made of, for example, metal or resin that covers a plurality of inner components of the distal end portion 3a.

In the cover member 10, a plurality of inner components are provided fixed by an adhesive 20 or the like.

To be more specific, as shown in Fig. 3, a plurality of lenses 12 supported by a lens frame 13 are provided on the distal end side in the cover member 10 so that the lens located on the outermost distal end side is exposed from the front end face of the distal end portion 3a.

Furthermore, the outer circumference of an element frame 14 on the distal end side is fixed to the outer circumference of the lens frame 13 on the proximal end side (opposite to the distal end).

A prism 15 is fixed inside the element frame 14 on the proximal end side. Furthermore, the image pickup device 16 is fixed to a light exit surface of the prism 15.

Thus, an image of the interior of the object introduced into the plurality of lenses 12 is introduced into the image pickup device 16 via the prism 15, where the image is picked up.

An electric substrate 17 is electrically connected to the image pickup device 16 and the distal end of a signal cable 21 inserted in the universal cord 5, the operation section 4 and the insertion portion 3 is electrically connected to the image pickup device 16 from the main unit 6 via the electric substrate 17.

A region in which the distal end of the signal cable 21 and the electric substrate 17 are fixed is covered with a filler 19 such as resin.

Thus, the image of the object picked up by the image pickup device 16 is transmitted to the main unit 6 via the signal cable 21, subjected to image processing by the CPU in the main unit 6 and then displayed on the monitor 6a as an endoscope image.

Furthermore, the distal end side of the light guide 18 inserted in the insertion portion 3 that transmits the light emitted from a light-emitting element provided in the operation section 4 to the front end face of the distal end portion 3a is also inserted in the cover member 10.

As shown in Fig. 3, the bending portion 3b is located behind the distal end portion 3a in an insertion direction S and the principal section thereof is configured by including a bent tube 32 in which a plurality of bending pieces are connected together along the insertion direction S and a soft tube member 30 made of, for example, rubber to cover the outer circumference of the bent tube 32.

In the bent tube 32, a bending piece 32f located in a region on the distal end side, that is, at a position closest to the distal end side among a plurality of bending pieces is engaged with and fixed to the inside of a proximal end 10k of the cover member 10 via an adhesive or the like.

Hereinafter, the position at which the bending piece 32f is engaged with the interior of the proximal end 10k of the cover member 10 will be referred to as "engagement region K."

Furthermore, a distal end 30s of the soft tube member 30 is fixed to an outer circumference of the bending piece 32f with a bobbin adhesion 31 in contact with the proximal end 10k of the cover member 10 without being adhered thereto.

Therefore, in the configuration of the present embodiment, as shown in Fig. 2 and Fig. 3, since the distal end 30s of the soft tube member 30 does not cover the outer circumference of the proximal end 10k of the cover member 10 as in the case of the conventional configuration, the cover member 10 and the soft tube member 30 have substantially the same diameter so that there is no difference in height between the cover member 10 and the soft tube member 30 in the insertion direction S.

Furthermore, as shown in Fig. 3, a puller wire 50 which is inserted in the insertion portion 3 so as to bend the bending portion 3b in upward and downward directions and bent into a loop shape at its distal end side is provided inside the bending portion 3b.

The loop-shaped puller wire 50 on the proximal end side is wound around a pulley (not shown) or the like provided inside the operation section 4.

Next, the fixing structure of the puller wire 50 on the distal end side will be described.

As shown in Fig. 2 to Fig. 4, a bottomed groove 11 having a predetermined length, for example, a length equal to or greater than half the perimeter along a circumferential direction C of the cover member 10 is formed in the vicinity of the proximal end 10k on an outer circumferential face 10g of the cover member 10.

Furthermore, as shown in Fig. 3 and Fig. 4, holes 25 and 26 communicating with the interior of the cover member 10 are formed in at least two places in the circumferential direction C of the groove 11, for example, at the bottom of one end 11i and another end 11t. The holes 25 and 26 are not limited to the one end 11i and the other end 11t, and may be formed at any positions as long as they are within the groove 11.

As shown in Fig. 2 and Fig. 4, the loop-shaped puller wire 50 on the distal end side is wound around the groove 11 from the inside of the cover member 10 through the holes 25 and 26.

To be more specific, the puller wire 50 is wound in such a way that it enters the groove 11 from the inside of the cover member 10 through the hole 25 and enters the cover member 10 from the groove 11 through the hole 26.

The puller wire 50 is formed to have an outside diameter equal to or smaller than the depth of the groove 11 so that when wound around the bottom of the groove 11, part of the puller wire 50 never sticks out of the groove 11 in a diameter direction Q of the cover member 10. In other words, the depth of the groove 11 is formed to be equal to or greater than the outside diameter of the puller wire 50.

This is because if part of the puller wire 50 sticks out of the groove 11, that is, from the outer circumferential face 10g of the cover member 10 in the diameter direction Q when the puller wire 50 is wound around the groove 11, the diameter of the distal end portion 3a increases by an amount corresponding to the portion of the puller wire 50 that sticks out.

Furthermore, for the purpose of securing watertightness and for the purpose of preventing the puller wire 50 from sticking out of the groove 11, the puller wire 50 when wound around the groove 11 is fixed by means of an adhesive 20 which is a fixing member.

In Fig. 2, the adhesive 20 is omitted for simplicity of the drawing. Furthermore, the fixing member is not limited to the adhesive 20, but may also be welding, brazing, solder or the like. Furthermore, the adhesive 20 is not only injected into the groove 11 but also the holes 25 and 26.

This prevents the position of the puller wire 50 wound around the groove 11 from moving and also secures watertightness.

A case of the aforementioned bottomed groove 11 has been described as an example where it has a length equal to or greater than half the perimeter along the circumferential direction C of the cover member 10, but the bottomed groove 11 is not limited to this, and with the distal end side of the puller wire 50 wound around the groove 11, the puller wire 50 is only required not to stick out of the outer circumferential face 10g of the cover member 10 in the diameter direction Q, and therefore a groove may be provided, for example, on the entire perimeter of the outer circumferential face of the cover member 10.

In this case, after the puller wire 50 is wound, it is preferable to fix the puller wire 50 to the groove provided on the entire perimeter of the cover member 10 using a fixing member such as an adhesive to secure watertightness and prevent the puller wire 50 from sticking out.

As described above, the puller wire 50 on the distal end side is wound around the groove 11 formed on the outer circumferential face 10g of the cover member 10 and fixed.

Thus, when the operator pulls the puller wire 50 in the upper part in Fig. 3 by operating the operation section 4, the bending portion 3b is bent in the upward direction, whereas when the operator pulls the puller wire 50 in the lower part in Fig. 3, the bending portion 3b is bent in the downward direction.

Here, the holes 25 and 26 are located forward of the engagement region K of the bending piece 3 2f in the proximal end 10k of the aforementioned cover member 10 in the insertion direction S.

This is because if the holes 25 and 26 are located behind the engagement region K, the puller wire 50 on the distal end side needs to be fixed to the bent tube 32 or the soft tube member 30. Furthermore, the holes 25 and 26 need to be formed in the bent tube 32 or the soft tube member 30. As described above, if the distal end side of the puller wire 50 is fixed to the bent tube 32, the problem is that the inner space of the bent tube becomes narrower. Furthermore, it is difficult to wind the puller wire 50 on the distal end side around the outer circumferential face of the soft tube member 30 and fix it from the standpoint of securing fixing strength.

Furthermore, as shown in Fig. 3, the holes 25 and 26 are formed inclined in the cover member 10 in such a way that openings 25ha and 26ha on the groove 11 side are located forward (direction shown by arrow A) of openings 25hb and 26hb inside the cover member 10 in the insertion direction S.

This is because if the holes 25 and 26 are formed to be perpendicular to the insertion direction S (direction shown by arrow A), the puller wire 50 that enters the holes 25 and 26 through the openings 25hb and 26hb needs to be bent by 90°, which may increase damage to the puller wire 50.

On the other hand, if the holes 25 and 26 are inclined forward in the insertion direction S, the bending angle of the puller wire 50 that enters the holes 25 and 26 through the openings 25hb and 26hb can be an obtuse angle, which can reduce damage to the puller wire 50 during bending.

Here, as shown in Fig. 3, the puller wire 50 is supported by wire receiving members 33 formed on a plurality of bending pieces by being shifted by 180° in the circumferential direction C in the bending portion 3b, but the openings 25hb and 26hb of the holes 25 and 26 are located coaxially with the wire receiving member 33 in the insertion direction S. That is, the positions of the openings 25hb and 26hb coincide with the positions of the wire receiving members 33 in the circumferential direction C.

This is intended to prevent the puller wire 50 from being twisted when the puller wire 50 supported by the wire receiving members 33 enters the holes 25 and 26 through the openings 25hb and 26hb.

Thus, the present embodiment has shown that in the bent tube 32, the bending piece 32f is engaged with and fixed to the proximal end 10k of the cover member 10 via an adhesive or the like and the distal end 30s of the soft tube member 30 is fixed to the outer circumference of the bending piece 32f by the bobbin adhesion 31, while being in contact with the proximal end 10k of the cover member 10.

Furthermore, it has been shown that the puller wire 50 on the distal end side is wound around the groove 11 formed on the outer circumferential face 10g of the cover member 10 through the holes 25 and 26, and fixed with the adhesive 20 so that watertightness is secured.

This eliminates the necessity for covering the distal end 30s of the soft tube member 30 on the outer circumference of the proximal end 10k of the cover member 10 to secure watertightness as in the case of the conventional configuration, and therefore the cover member 10 and the soft tube member 30 have substantially the same diameter and no difference in height is produced between the cover member 10 and the soft tube member 30 in the insertion direction S.

This makes it possible to reduce the outside diameter of the insertion portion 3 on the distal end side and thereby improves insertability of the insertion portion 3 into the object.

Moreover, since the distal end 30s of the soft tube member 30 need not be covered on the outer circumference of the proximal end 10k of the cover member 10, it is not necessary to fix the distal end of the soft tube member 30 at the position of the outer circumferential face 10g of the cover member 10 other than the position of the groove 11 as in the case of the conventional configuration, and the length of the distal end portion 3a can thereby be shortened and insertability of the insertion portion 3 into the object improves.

Furthermore, the puller wire 50 on the distal end side is not fixed to the bent tube 32 as in the case of the conventional art, but it is fixed to the groove 11 formed on the outer circumferential face 10g of the cover member 10, which avoids the inner space of the proximal end 10k of the cover member 10 and the bending portion 3b from being narrowed.

Furthermore, the puller wire 50 on the distal end side can be fixed to the groove 11 with only the adhesive 20, which eliminates the necessity for securing the thickness with which the screw for fixing the puller wire 50 is fixed in the cover member 10 as in the case of the conventional art and thereby prevents the inner space of the cover member 10 on the proximal end side from being narrowed, and can also easily prevent the cover member 10 from dropping by fixing the puller wire 50 to the groove 11.

Furthermore, the proximal end of the cover member where the soft tube member on the distal end side is covered need not be placed inward in the diameter direction Q, so that the cover member 10 and the soft tube member 30 have the same diameter as in the case of the conventional art, and it is thereby possible to secure a wide inner space of the cover member 10 on the proximal end side.

Therefore, it is possible to secure a wide inner space in the proximal end 10k of the cover member 10 and the bending portion 3b, thereby increase the number of light guides 18 to increase light quantity or increase the thickness of the signal cable 21 and thereby reduce image noise.

As described so far, it is possible to provide the endoscope 2 having a configuration capable of shortening the distal end portion 3a while reducing the diameter of the insertion portion 3 on the distal end side and securing sufficient inner space.

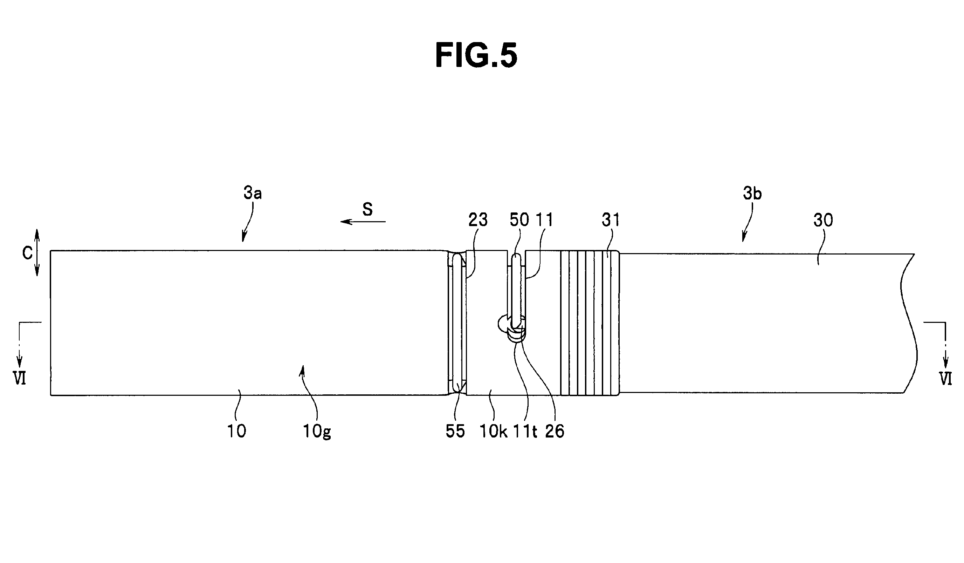

Hereinafter, modification examples will be illustrated using Fig. 5 to Fig. 7.

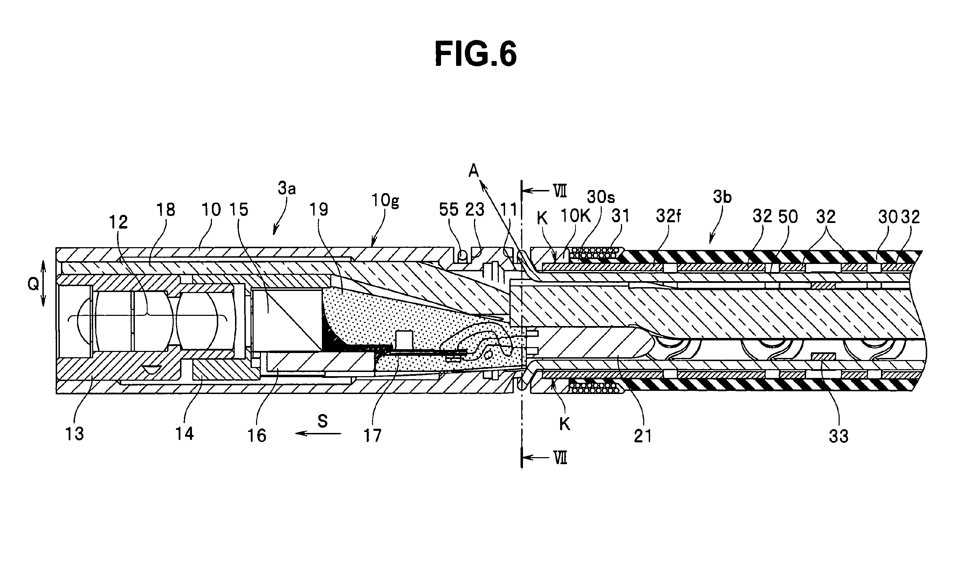

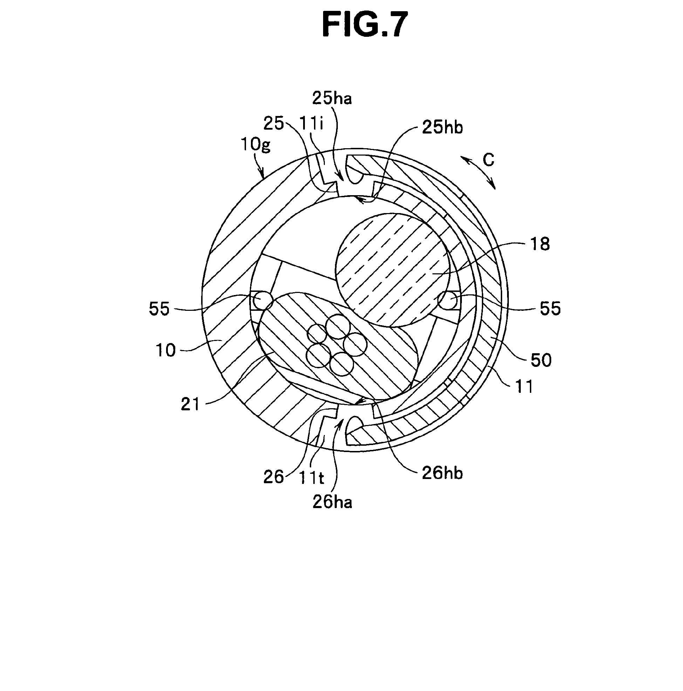

Fig. 5 is an enlarged perspective view of a configuration of a modification example of the insertion portion of the endoscope on the distal end side in Fig. 2, Fig. 6 is a cross-sectional view of the insertion portion on the distal end side along a line VI-VI in Fig. 5 and Fig. 7 is a cross-sectional view of the distal end portion along a line VII-VII in Fig. 6.

The aforementioned present embodiment has shown that the bending portion 3b is bendable in two directions; upward and downward, and one puller wire 50 is provided in the bending portion 3b, which is inserted in the insertion portion 3 and bent into a loop shape on the distal end side so as to bend the bending portion 3b in the upward and downward directions.

Without being limited to this, however, the bending portion 3b may also be bendable in two or more directions.

Hereinafter, a configuration in which the bending portion 3b is bendable in, for example, four directions; up, down, left and right, will be described using Fig. 5 to Fig. 7.

In Fig. 5 and Fig. 7, the adhesive 20 is omitted for simplicity of the drawings.

As shown in Fig. 6 and Fig. 7, in addition to the puller wire 50 for upward/downward bending which is bent into a loop shape, a puller wire 55 for leftward/rightward bending which is bent into a loop shape is inserted at a position shifted by 90° in the circumferential direction C with respect to, for example, the puller wire 50 in the insertion portion 3.

Like the puller wire 50 on the proximal end side, the puller wire 55 on the proximal end side is also wound around another pulley which is different from the pulley wound with the puller wire 50 provided in the operation section 4.

Furthermore, as shown in Fig. 5 and Fig. 6, a bottomed groove 23 having a predetermined length, for example, a length equal to or greater than half the perimeter is formed forward of the groove 11 on the outer circumferential face 10g of the cover member 10 along the circumferential direction C. The groove 23 is shifted by 90° in the circumferential direction C with respect to the groove 11.

Furthermore, holes (not shown) communicating with the interior of the cover member 10 are formed at the bottom of the groove 23 at one end and the other end in the circumferential direction C at a position shifted, for example, by 90° from the one end 11i and the other end 11t of the groove 11 in the circumferential direction C.

The distal end side of the loop-shaped puller wire 55 is wound around the groove 23 from the inside of the cover member 10 through the holes at one end and the other end of the groove 23 as shown in Fig. 5 and Fig. 6.

To be more specific, the puller wire 55 is wound in such a way that the puller wire 55 enters the groove 23 from the inside of the cover member 10 through the hole at the one end and enters the cover member 10 from the groove 23 through the hole at the other end.

Like the puller wire 50, the puller wire 55 is formed to have an outside diameter equal to or smaller than the depth of the groove 23 so that when wound around the bottom of the groove 23, part of the puller wire 55 never sticks out of the groove 23 in the diameter direction Q of the cover member 10.

Furthermore, for the purpose of securing watertightness and for the purpose of preventing the puller wire 55 from sticking out of the groove 23, the puller wire 55 when wound around the groove 23 is fixed by means of the aforementioned fixing member (not shown) in the same way as the puller wire 50.

The fixing member is naturally also set in the holes formed in the groove 23.

This prevents the position of the puller wire 55 wound around the groove 23 from moving and also secures watertightness.

As described above, the puller wire 55 on the distal end side is wound around and fixed to the groove 23 which is different from the groove 11 on the outer circumferential face 10g of the cover member 10.

Thus, when the operator pulls the puller wire 55 on the left side in Fig. 7 by operating the operation section 4, the bending portion 3b is bent leftward, and when the operator pulls the puller wire 55 on the right side, the bending portion 3b is bent rightward.

Since the shape of the holes formed in the groove 23 is the same as the shape of the holes 25 and 26 formed in the groove 11, the description thereof will be omitted.

As described above, a number of grooves corresponding to the puller wires may be formed at positions shifted in the insertion direction S respectively on the outer circumferential face 10g of the cover member 10.

Therefore, when three or more loop-shaped puller wires are inserted in the insertion portion 3, a number of grooves corresponding to the number of puller wires may be shifted in the insertion direction S on the outer circumferential face 10g of the cover member 10.

According to such a configuration, even when the endoscope 2 has the bending portion 3b which is bent in two or more directions, it is possible to obtain effects similar to those of the present embodiment.

### (Second embodiment)

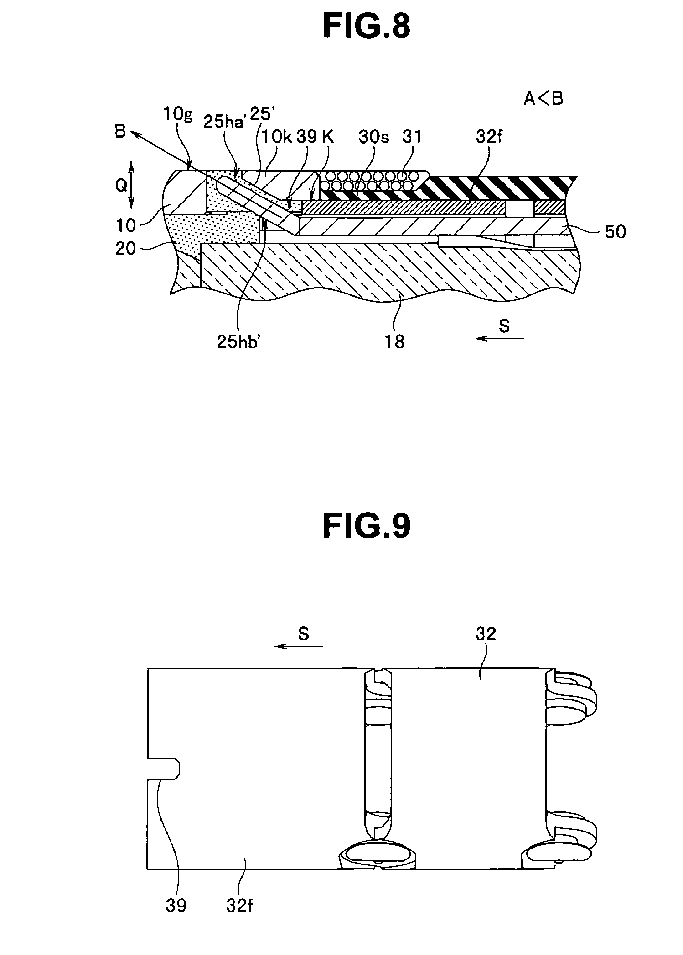

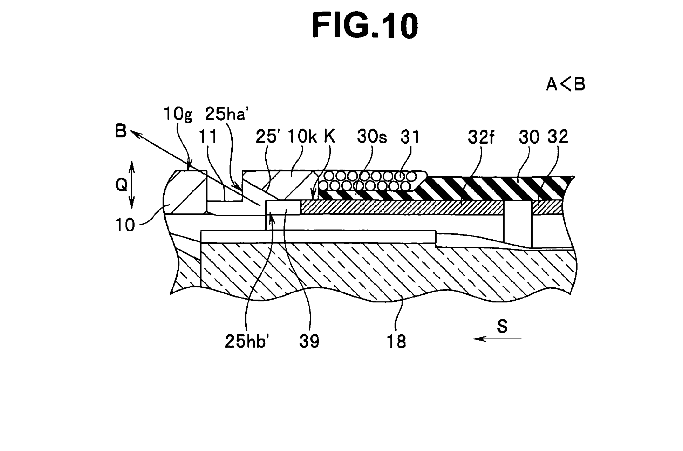

Fig. 8 is an enlarged partial cross-sectional view illustrating the engagement region between the distal end portion and the bending portion in the insertion portion of an endoscope according to a second embodiment, Fig. 9 is an enlarged plan view of the distal end side of the bent tube in Fig. 8 and Fig. 10 is a partial cross-sectional view of the engagement region with the puller wire and adhesive omitted from Fig. 8.

The configuration of the endoscope of this second embodiment is different from the endoscope of the first embodiment shown in aforementioned Fig. 1 to Fig. 7 in that the opening inside the cover member in the holes formed at one end and the other end of the groove of the cover member is located in the region in which the bent tube on the distal end side is engaged with the proximal end of the cover member.

Therefore, only the difference will be described and the same components as those of the first embodiment will be assigned the same reference numerals and the description thereof will be omitted.

As shown in Fig. 8 and Fig. 10, in the holes 25 and 26 at one end 11i and the other end 11t of the groove 11 in the circumferential direction C formed on the outer circumferential face 10g of the cover member 10 according to the present embodiment, the openings 25hb and 26hb inside the cover member 10 (opening 26hb is not shown) are located in the engagement region K of the bending piece 32f in the proximal end 10k of the cover member 10.

That is, holes 25' and 26' (hole 26' is not shown) of the present embodiment are greatly inclined with respect to the outer circumferential face 10g of the cover member 10 in a direction shown by arrow B (A<B) in Fig. 8 and Fig. 10 compared to the holes 25 and 26 of the aforementioned first embodiment.

Furthermore, as shown in Fig. 9 and Fig. 10, a slit 39 which communicates with the holes 25' and 26', and which is along the insertion direction S in which the puller wire 50 is inserted as shown in Fig. 8 is formed on the bending piece 32f on the distal end side located in the engagement region K.

The rest of the configuration is the same as that of the aforementioned first embodiment.

According to such a configuration, since the puller wire 50 is inserted into the slit 39, the bending piece 32f never rotates in the circumferential direction C, and it is thereby possible to easily position the puller wire 50 and the bent tube 32 in the circumferential direction C during assembly and improve the accuracy of assembly.

Furthermore, since the holes 25' and 26' are greatly inclined with respect to the outer circumferential face 10g of the cover member 10 more than the holes 25 and 26 of the first embodiment, the bending angle of the puller wire 50 that enters the holes 25' and 26' through the openings 25hb' and 26hb' can be a more obtuse angle than the first embodiment and damage to the puller wire 50 during bending can be made smaller than the first embodiment.

Furthermore, since the slit 39 communicates with the holes 25' and 26', that is, the bending piece 32f can be engaged forward by an amount corresponding to the length of the slit 39 in the insertion direction S, and it is thereby possible to shorten the rear end 10k of the cover member 10 and shorten the length of the distal end portion 3a in the insertion direction S by an amount corresponding to the length of the slit 39.

Other effects are similar to those of the aforementioned first embodiment.

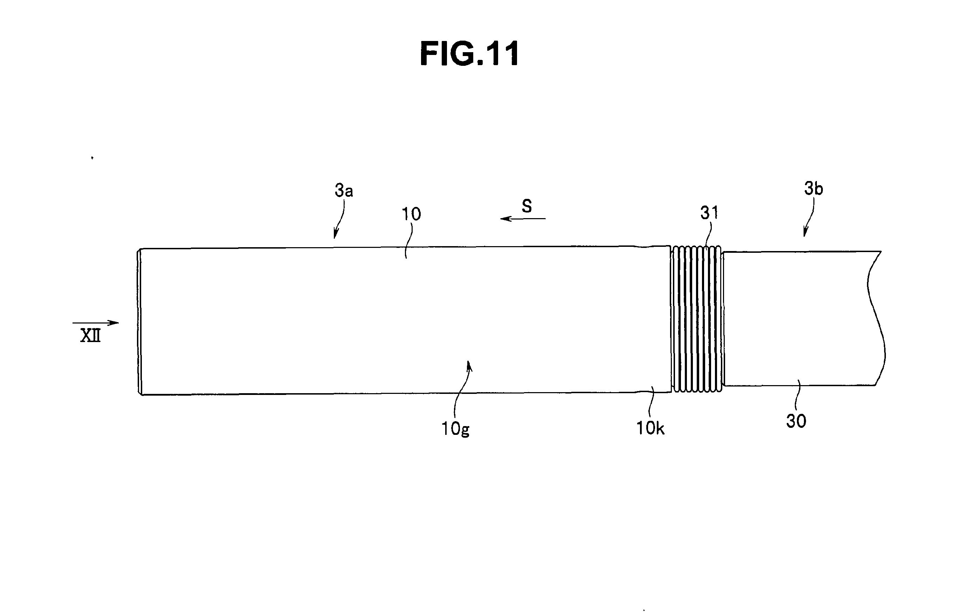

Hereinafter, modification examples will be described using Fig. 11 to Fig. 14.

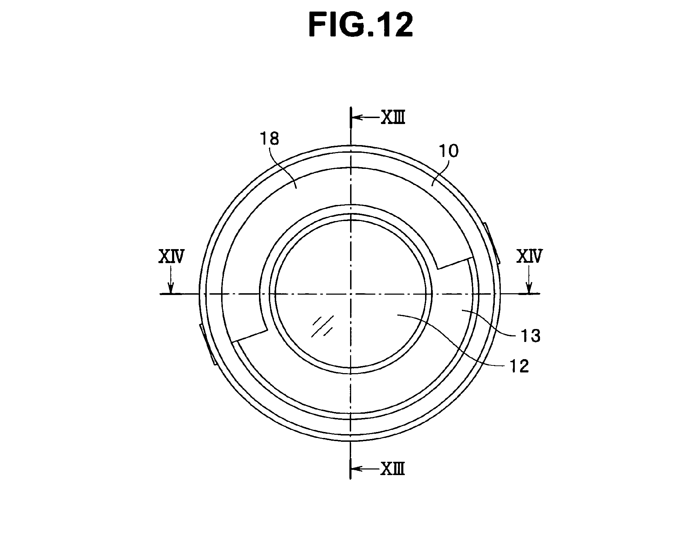

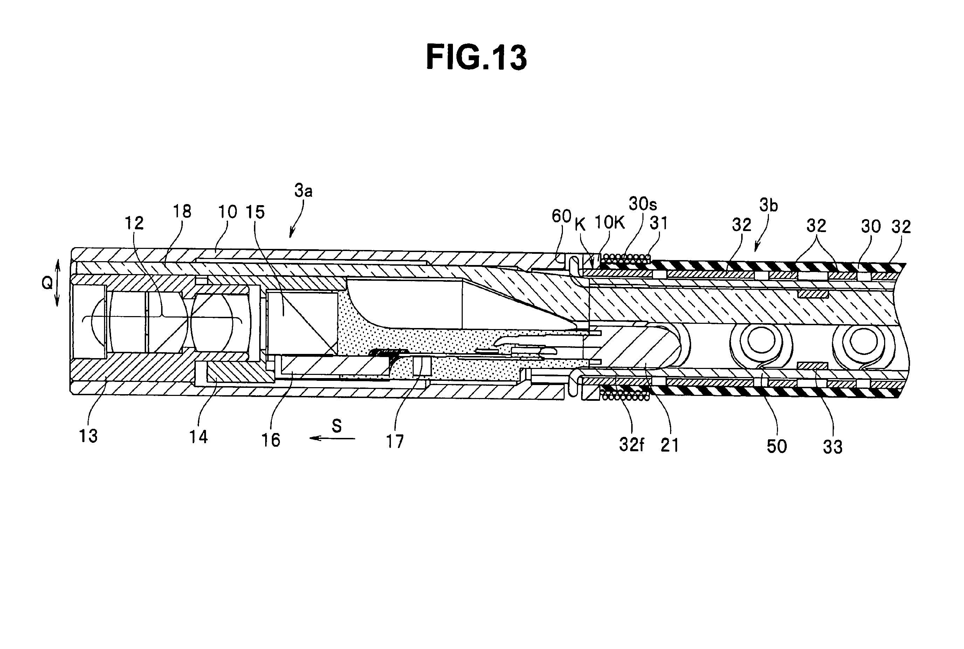

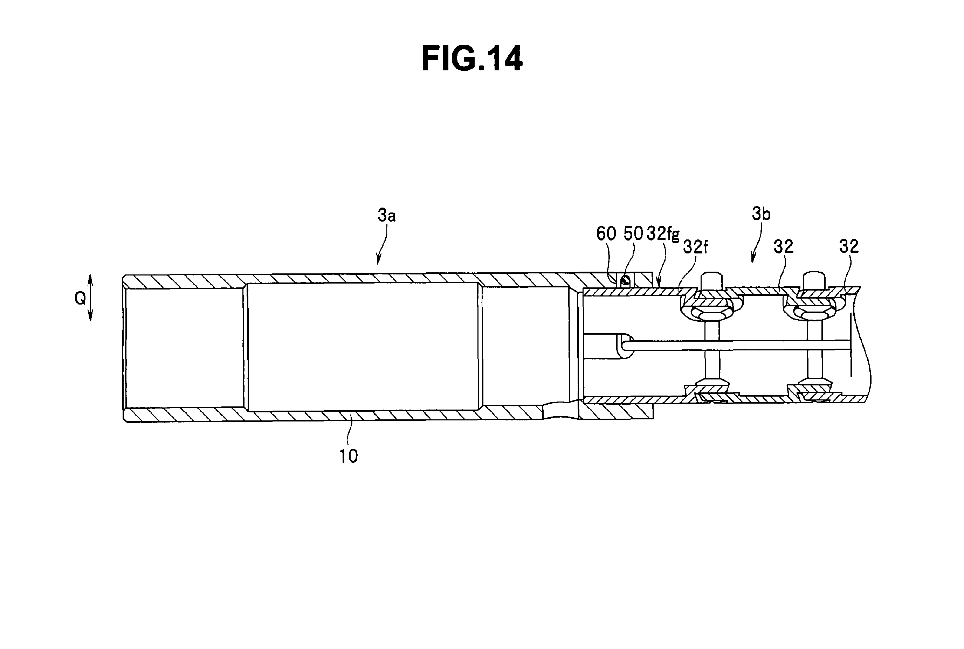

Fig. 11 is an enlarged perspective view illustrating the insertion portion on the distal end side in a configuration according to a modification example where a puller wire is wound around the bent tube, Fig. 12 is a side view of the distal end portion in Fig. 11 viewed from a direction XII in Fig. 12, Fig. 13 is a cross-sectional view of the insertion portion on the distal end side along a line XIII-XIII in Fig. 12 and Fig. 14 is a cross-sectional view of the insertion portion on the distal end side along a line XIV-XIV in Fig. 12.

The aforementioned first and second embodiments have shown that the puller wire 50 which bends the bending portion 3b is wound around and fixed to the groove of the outer circumferential face 10g of the cover member 10.

Without being limited to this, as shown in Fig. 11 to Fig. 14, the present invention may also adopt a configuration in which the puller wire 50 is wound around an outer circumferential face 32fg of the bending piece 32f and a slit 60 in which the puller wire 50 is set is provided at the proximal end 10k of the cover member 10 that covers the outer circumferential face 32fg.

According to such a configuration, since the puller wire 50 is wound around the outer circumferential face 32fg of the bending piece 32f, the inner space is never narrowed, and moreover, although the puller wire 50 is wound around the outer circumferential face 32fg of the bending piece 32f, since the puller wire 50 is set in the slit 60 of the proximal end 10k of the cover member 10, the puller wire 50 never causes the cover member 10 to be widened toward the outside in the diameter direction Q. That is, the cover member 10 never increases in the diameter direction Q.

Thus, as shown in Fig. 11, according to the present configuration, the puller wire 50 wound around the outer circumferential face 32fg of the bending piece 32f is never exposed from the outer circumferential face 10g of the cover member 10.

Furthermore, since the slit 60 is hooked at the puller wire 50, it is possible to easily prevent the cover member 10 from dropping.

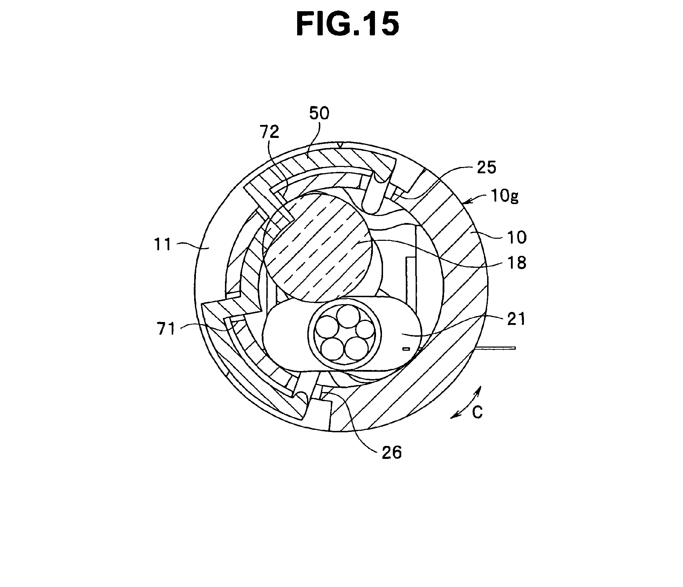

Furthermore, another modification example will be shown below using Fig. 15.

Fig. 15 is a cross-sectional view illustrating a modification example where a puller wire is set in a plurality of holes of the groove of the cover member in Fig. 4 in a running stitch form.

The aforementioned first and second embodiments have shown that the puller wire 50 is wound in such a way that the puller wire 50 enters the groove 11 from the cover member 10 through the hole 25 and enters the cover member 10 from the groove 11 through the hole 26.

Without being limited to this, a configuration may also be adopted in which as shown in Fig. 15, in addition to the holes 25 and 26, holes communicating with the interior of the cover member 10 are provided in regions from one end 11i to the other end 11t of the groove 11 in the circumferential direction C as a plurality of holes 71 and 72, and the puller wire 50 is wound around the groove 11 with the puller wire 50 set in the holes 25, 71, 72 and 26 in a running stitch form.

According to such a configuration, it is possible to fix the puller wire 50 to the groove 11 more strongly than the aforementioned first and second embodiments. It goes without saying that the number of holes formed between the holes 25 and 26 is not limited to two.

The present invention is not limited only to the above-described embodiments, but can be implemented modified in various ways without departing from the spirit and scope of the present invention.

## Claims

1. An endoscope comprising:
a cover member that constitutes a distal end portion on a distal end side in an insertion direction of an insertion portion inserted into an object and covers inner components in the distal end portion;
a groove formed along a circumferential direction of the cover member on an outer circumferential face of the cover member;
holes located in at least two places in the circumferential direction of the groove, communicating with an interior of the cover member and formed in the cover member;
a bending portion located behind the distal end portion in the insertion direction of the insertion portion, comprising a bent tube and a soft tube member for coating the bent tube in which the distal end side of the bent tube in the insertion direction is engaged within a proximal end of the cover member in the insertion direction and the distal end of the soft tube member in the insertion direction contacts the proximal end of the cover member; and
a puller wire that has an outside diameter equal to or smaller than a depth of the groove, is inserted into the insertion portion so as to bend the bending portion in a plurality of directions, enters the groove from the interior of the cover member through one of the holes of the cover member, is folded into a loop shape so as to enter the cover member through the other hole from the groove and is wound around the groove.

2. The endoscope according to claim 1, wherein the puller wire is fixed to the groove using a fixing member so that the puller wire does not stick out of the outer circumferential face of the cover member in a diameter direction of the cover member.

3. The endoscope according to claim 1 or 2, wherein the one and the other holes formed in the groove are located forward of a region in which the bent tube on the distal end side is engaged with the proximal end of the cover member in the insertion direction.

4. The endoscope according to any one of claims 1 to 3, wherein the one and the other holes are formed inclined in the cover member so that an opening on the groove side is located forward of an opening inside the cover member in the insertion direction.

5. The endoscope according to any one of claims 1 to 4, wherein the puller wire is supported in the bent tube by a wire receiving member provided in the bent tube, and
an opening inside the cover member in the one and the other holes is provided coaxially with the wire receiving member in the insertion direction.

6. The endoscope according to any one of claims 1 to 5, wherein the puller wire is inserted in plurality in the insertion portion, and
a number of the grooves corresponding to the puller wires are formed on the outer circumferential face of the cover member.

7. The endoscope according to any one of claims 1 to 6, wherein a plurality of the holes are further formed in the groove between the one hole and the other hole in the circumferential direction, and
the puller wire is wound around the groove with the puller wire set in a running stitch form from the one hole to the other hole through the plurality of holes and wound around the groove.

8. The endoscope according to any one of claims 1 to 7, wherein openings inside the cover member in the one and the other holes are located in a region in which the bent tube on the distal end side is engaged with the proximal end of the cover member, and
a slit which communicates with the holes and into which the puller wire is inserted is formed on the distal end side of the bent tube located in the engagement region.
